# EUROPÄISCHE PATENTSCHRIFT

(11) **EP 2 349 117 B1**
(45) Veröffentlichungstag und Bekanntmachung des Hinweises auf die Patenterteilung: **06.03.2019**
(21) Anmeldenummer: 09783380.0
(22) Anmeldetag: 24.09.2009
(51) Int. Cl.: A61F 2/46, A61F 2/00, A61F 2/30

(54) **INSTRUMENT ZUR HANDHABUNG EINER GELENKKOMPONENTE MITTELS UNTERDRUCK**
INSTRUMENT FOR HANDLING A JOINT COMPONENT BY WAY OF A VACUUM
INSTRUMENT POUR MANIPULER UN COMPOSANT D'ARTICULATION, PAR DÉPRESSION

(30) Priorität: 30.09.2008 DE 102008049661
(43) Veröffentlichungstag der Anmeldung: 03.08.2011
(73) Patentinhaber: Smith & Nephew Orthopaedics AG, 6343 Rotkreuz (CH)
(72) Erfinder: IMHOF-RÖTHLIN, Martin, CH-6045 Meggen (CH); BRACK, René, "verstorben" (CH)
(74) Vertreter: Popp, Eugen
(86) Internationale Anmeldenummer: PCT/EP2009/062394
(87) Internationale Veröffentlichungsnummer: WO 2010/037685

(56) Entgegenhaltungen:
- WO-A-2007/025639
- DE-A1- 10 128 234
- DE-A1- 19 704 577
- FR-A- 2 877 210
- US-A1- 2005 137 603
- US-A1- 2007 225 725

## Beschreibung

Die vorliegende Erfindung betrifft ein Instrument zur Handhabung einer eine, insbesondere konkave, Gelenkfläche umfassenden Gelenkkomponente einer Gelenkprothese, insbesondere Hüft- oder Schulterpfanneninlay-Setzinstrument, mit einem Griffteil und einem an die Gelenkfläche abdichtend anlegbaren Saugelement.

Derartige Instrumente sind allgemein bekannt. Es wird diesbezüglich nur beispielhaft verwiesen auf die DE 197 04 577 A1, DE 101 28 234 A1 oder WO 2007/025639 A1, ferner auf die FR 2877210.

All diesen vorbekannten Setzinstrumenten ist ein Saugelement in Form eines Saugnapfes gemeinsam. In der Praxis haben sich diese Setzinstrumente durchaus bewährt. Sie sind jedoch handhabungstechnisch relativ aufwendig und auch nur bedingt funktionssicher. Beim Stand der Technik gemäß der DE 101 28 234 A1 und DE 197 22 923 A1 kommt noch hinzu, daß dort jeweils eine Zweihand-Bedienung zwingend notwendig ist. Auch besteht jeweils die Gefahr, daß ungewollt der Unterdruck im Saugraum zwischen Saugnapf und Gelenkfläche aufgehoben wird mit der Folge, daß die Gelenkkomponente in einem unerwünschten Moment vom Instrument fällt. Der Grund dafür liegt z.B. bei dem Setzinstrument gemäß der DE 101 28 234 A1 vornehmlich darin, daß das Betätigungselement für das Druckentlastungsventil axial über die Grifffläche nach hinten vorsteht, d.h. in Richtung zum Benutzer hin. Somit kann nicht ausgeschlossen werden, daß beim Greifen des Instruments versehentlich das Betätigungselement für das Druckentlastungsventil berührt und unerwünscht betätigt wird.

Der vorliegenden Erfindung liegt also ausgehend vom erwähnten Stand der Technik die Aufgabe zugrunde, ein Instrument der eingangs genannten Art zu schaffen, welches eine Einhand-Bedienung und hohe Funktionssicherheit gewährleistet, und welches insbesondere auch intraoperativ eingesetzt werden kann.

Diese Aufgabe wird erfindungsgemäß durch die kennzeichnenden Maßnahmen des Anspruches 1 gelöst, wobei vorteilhafte Weiterentwicklungen und konstruktive Details in den Unteransprüchen beschrieben sind.

Die vorliegende Erfindung bezieht sich auch auf eine Anordnung für ein Setzinstrument der hier fraglichen Art gemäß Anspruch 15. In der Praxis werden nämlich Gelenkkomponenten unterschiedlicher Größe eingesetzt. In Anpassung daran soll erfindungsgemäß ein Satz von weichelastischen Formteilen unterschiedlicher Größe zur Verfügung gestellt werden, ohne daß im übrigen das Setzinstrument abgeändert werden müßte.

Das erfindungsgemäße Instrument basiert auf dem Prinzip des Unterdrucks. Es umfaßt ein Saugelement mit einem der Gelenkfläche angepaßten weichelastischen Formteil, welches einen Abschnitt aufweist, dessen Oberfläche der zugeordneten Gelenkfläche möglichst genau angepaßt ist. Damit entsteht bereits beim Zusammenfügen von Saugelement und Gelenkfläche ein nicht unbeachtliches Vakuum. Dieses Vakuum gilt es jedoch zu verstärken. Zu diesem Zweck ist dem weichelastischen Formteil ein Zugelement zugeordnet, durch welches das weichelastische Formteil so verformbar sein soll, daß bei Anlage an der Gelenkfläche zwischen dieser und dem Formteil ein erhöhter Unterdruck entsteht. Durch das Zugelement soll das Formteil an der Stelle, an der das Zugelement angreift, in das Formteil hinein verformt werden. Dadurch wird das Instrument in das Implantat regelrecht hineingezogen. Gleichzeitig erfährt der Bereich um den Angriffspunkt des Zugelements herum eine radiale Ausdehnung, wodurch eine erhöhte Abdichtung zwischen weichelastischem Formteil und Gelenkfläche entsteht. Dadurch wird insgesamt eine hohe Funktionssicherheit bei der Handhabung des erfindungsgemäßen Instrumentes erreicht. Die Gefahr, daß die Gelenkkomponente versehentlich vom Setzinstrument herabfällt, besteht nicht. Das erfindungsgemäße Instrument läßt sich auch gut zur exakten Navigation des Implantates verwenden. Aufgrund des hohen Befestigungsgrades der Gelenkkomponente am Instrument eignet sich dieses auch für eine intraoperative Handhabung, d.h. Entfernung der Gelenkkomponente und neue Ausrichtung derselben während der Operation. Das erfindungsgemäße Instrument eignet sich insbesondere zur Handhabung einer Zementpfanne oder eines Pfanneneinsatzes aus Polyethylen, Metall oder Keramik, d.h. als Hüft- oder Schulterpfanneninlay-Setzinstrument.

Das weichelastische Formteil besteht vorzugsweise aus Silikon.

Als Zugelement hat sich bei einer bevorzugten Ausführungsform eine Zugschraube mit Gewindeschaft und Schraubenkopf bewährt, wobei bei hemisphärischem Saugabschnitt des weichelastischen Formteils der Schraubenkopf dann vorzugsweise dem Pol des hemisphärischen Saugabschnitt zugeordnet ist. Der Gewindeschaft der Zugschraube ist in das distale Ende des stabartigen Griffteils einschraubbar, wobei dann das weichelastische Formteil zwischen Schraubenkopf und Griffteil bzw. einer am distalen Ende desselben angeordneten Stützfläche eingespannt ist.

Die Zugschraube kann auch über einen Drahtseilabschnitt oder dünnen Stab mit einem Betätigungsmittel am Griffteil gekoppelt sein, wobei das Betätigungsmittel zum Beispiel einen Exzenter umfassen kann.

Alternativ ist es auch denkbar, daß die Zugkraft auf das weichelastische Formteil durch eine externe Saugeinrichtung aufgebracht wird. Als Zugelement dient dann z.B. ein am distalen Ende des Griffteils verschieblich gelagerter Kolben, der über eine sich durch das Griffteil hindurch erstreckende Leitung nach innen gesaugt werden kann unter entsprechender Mitnahme und Verformung des weichelastischen Formteils.

Nachstehend wird jedoch eine besonders bevorzugte und konstruktiv einfache sowie sehr funktionssichere Ausführungsform eines erfindungsgemäßen Setzinstrumentes anhand der beigefügten Zeichnung näher erläutert. Diese zeigt in:
- Fig. 1: eine Ausführungsform eines erfindungsgemäßen Setzinstruments in perspektivischer Ansicht;
- Fig. 2: das Setzinstrument gemäß Fig. 1 in Seitenansicht, jedoch teilweise im Schnitt; und
- Fig. 3: das Setzinstrument gemäß den Figuren 1 und 2 in perspektivischer Explosionsdarstellung.

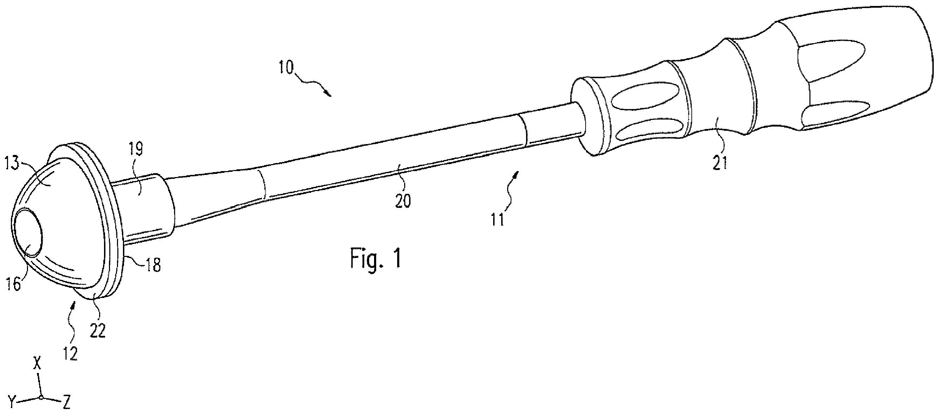

In den Figuren 1 bis 3 ist ein Hüft- oder Schulterpfanneninlay-Setzinstrument, im folgenden nur noch Setzinstrument genannt, dargestellt, welches ein Griffteil 11 und ein an die Gelenkfläche einer hier nicht dargestellten Gelenkprothese, z.B. Pfanne, abdichtend anlegbares Saugelement 12 aufweist. Das Saugelement 12 umfaßt ein der Gelenkfläche angepaßtes weichelastisches Formteil 13, welches vorzugsweise aus Silikon od. dgl. hergestellt ist, und welches durch ein Zugelement in Form einer Zugschraube 14 derart verformbar ist, daß bei Anlage an die Gelenkfläche zwischen dieser und dem weichelastischen Formteil 13 Unterdruck entsteht. Das Zugelement in Form der Zugschraube 14 ist vom Griffteil 11 her manipulierbar, worauf weiter unten noch näher eingegangen wird. Das weichelastische Formteil 13 umfaßt einen hemisphärischen Saugabschnitt in Anpassung an eine konkave Gelenkfläche eines Hüft- oder Schulterpfanneninlays aus Polyethylen, Keramik oder Metall.

Die Zugschraube 14 weist einen Gewindeschaft 15 und einen Schraubenkopf 16 auf. Dieser Schraubenkopf 16 ist bei der hemisphärischen Ausbildung des Saugabschnitts des weichelastischen Formteils 13 dem Pol desselben zugeordnet, wie insbesondere Fig. 1 sehr gut erkennen läßt.

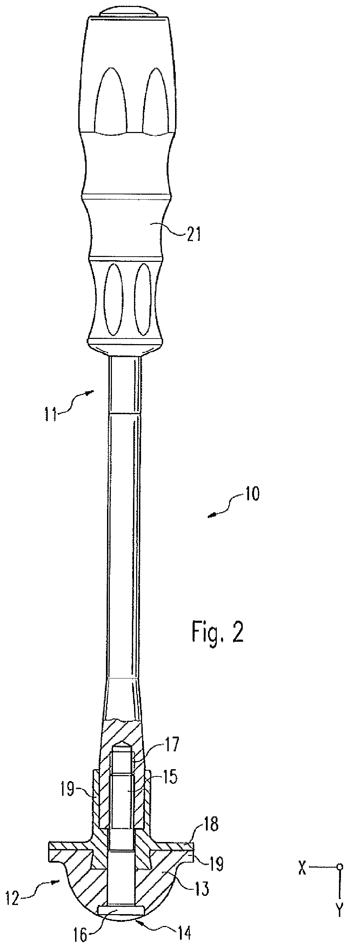

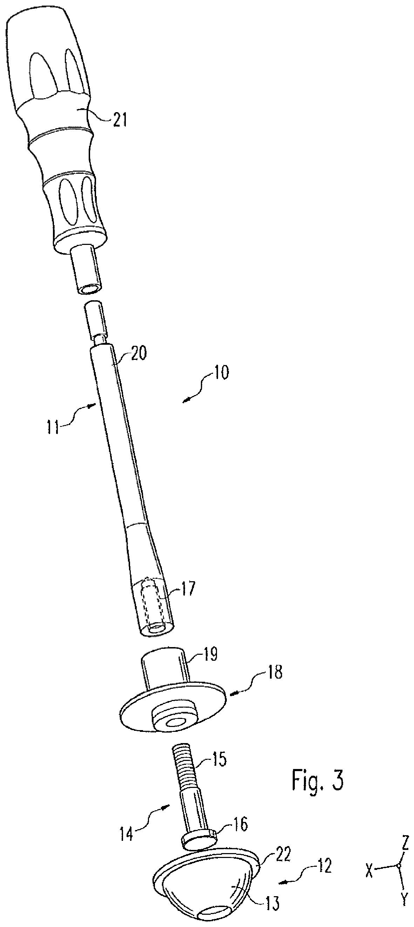

Der Gewindeschaft 15 der Zugschraube 14 ist, wie die Figuren 2 und 3 sehr deutlich zeigen, in das distale Ende des Griffteils 11 einschraubbar. Das am distalen Ende des Griffteils 11 vorgesehene Innengewinde ist in Fig. 3 mit der Bezugsziffer 17 gekennzeichnet. In dieses ist der Gewindeschaft 15 der erwähnten Zugschraube 14 einschraubbar. In diesem Fall ist dann das weichelastische Formteil 13 zwischen dem Schraubenkopf 16 und dem Griffteil 11 bzw. einer am distalen Ende desselben angeordneten Stützfläche eingespannt. Bei der dargestellten Ausführungsform wird die Stützfläche durch einen Stützflansch 18 definiert. Dieser Sützflansch 18 ist als gesondertes Bauteil auf das distale Ende des Griffteils 11 aufsteckbar. Zu diesem Zweck umfaßt der Stützflansch 18 eine Aufsteckhülse 19. In diese wird das distale Ende des Griffteils 11 gesteckt. Die Zuordnung zwischen Griffteil 11 und Stützflansch 18 samt Aufsteckhülse 19 wird durch die Zugschraube 14 sichergestellt.

Die Zugschraube 14, die sich im montierten Zustand des Setzinstruments 10 axial durch das weichelastische Formteil 13 hindurcherstreckt, ist innerhalb des weichelastischen Formteils 13 drehfest gesichert. Dadurch läßt sich durch Drehen des Griffteils 11 innerhalb der Aufsteckhülse 19 relativ zu dieser, dem Stützflansch und damit auch zum Saugelement 12 bzw. weichelastischen Formteil 13 die Zugschraube 14 mehr oder weniger weit in das distale Ende des Griffteils 11 bzw. in das dort vorgesehene Innengewinde 17 hineinschrauben mit der Folge, daß dann auch der Schraubenkopf 16 das weichelastische Formteil 13 im Polbereich desselben mehr oder weniger weit nach innen zieht. Dadurch entsteht ein erhöhter Unterdruck im Polbereich des weichelastischen Formteils 13 zwischen diesem und der zugeordneten Gelenkfläche. Gleichzeitig wird das weichelastische Formteil durch diese Verformung im Polbereich nach innen im Abstand vom Polbereich nach außen gedrängt, wodurch eine zusätzliche Pressung zwischen weichelastischem Formteil 13 und Gelenkfläche entsteht. Diese zusätzliche Pressung erhöht die Dichtwirkung zwischen weichelastischem Formteil 13 und Gelenkfläche sowie auch die Verdrehsicherheit zwischen weichelastischem Formteil 13 und Gelenkfläche.

Das Griffteil 11 weist, wie die Figuren 1 bis 3 sehr gut erkennen lassen, einen stabförmigen Abschnitt 20 auf, an dessen distalem Ende das weichelastische Formteil 13 abgestützt ist und dessen proximales Ende einen Handgriff 21 umfaßt. Dieser ist nach Art eines Schraubendreher-Griffs ausgebildet. Der stabartige Abschnitt 20 des Griffteils 11 ist bei der dargestellten Ausführungsform starr ausgebildet. Er kann jedoch zumindest bereichsweise auch biegeelastisch, z.B. nach Art einer flexiblen Antriebswelle, ausgebildet sein. Dadurch wird der Freiheitsgrad für den Operateur zusätzlich erhöht. Natürlich muß die Übertragung von Drehmomenten auf die Zugschraube gewährleistet sein.

Des weiteren lassen die Figuren 1 bis 3 gut erkennen, daß das weichelastische Formteil 13 einen sich äquatorial und radial nach außen erstreckenden Umfangsrand 22 aufweist. Mit diesem Umfangsrand läßt sich das weichelastische Formteil 13 am entsprechenden Umfangsrand einer Pfanne, insbesondere eines Pfanneninlays abstützen. Dadurch wird zusätzlich eine Verdrehsicherheit zwischen weichelastischem Formteil 13 und der Gelenkkomponente erzielt, insbesondere unmittelbar nach dem Einsetzen des noch unverformten Saugelements 12 bzw. weichelastischen Formteils 13 in die Gelenkkomponente.

Wie schon oben erwähnt, ist am distalen Ende des Griffteils 11, und zwar des stabförmigen Abschnitts 20 desselben ein Stützflansch 18 angeordnet, gegen den das weichelastische Formteil 13 abstützbar ist. Der Stützflansch 18 erstreckt sich über die gesamte proximale bzw. dem Griffteil 11 zugewandte Fläche des weichelastischen Formteils 13, also auch über den erwähnten Umfangsrand 22. Der Umfangsrand 22 wird also ebenfalls abgestützt.

Die verdrehsichere Verankerung der Zugschraube 14 innerhalb des weichelastischen Formteils 13 kann im übrigen auch noch dadurch erhalten werden, daß die Zugschraube 14 in das weichelastische Formteil 13 eingegossen ist. Bei modularer Ausbildung weist der Schaft zwischen Gewinde und Schraubenkopf vorzugsweise einen eckigen oder elliptischen Querschnitt auf, der mit einem entsprechenden Querschnitt eines axialen Durchgangs durch das weichelastische Formteil korrespondiert.

Im übrigen sind auch Maßnahmen getroffen, daß das weichelastische Formteil 13 gegenüber dem Stützflansch 18 verdrehsicher gehalten ist, so daß bei Drehen des Griffteils 11 um die Längsachse desselben dieses nur relativ zu den beiden vorgenannten Teilen 13, 18 und zur Zugschraube 14 verdrehbar ist. In diesem Fall dient die Aufsteckhülse 19 also als reine Führungshülse für das distale Ende des Griffteils 11.

Durch die Zugschraube 14 werden das weichelastische Formteil 13, der Stützflansch 18 samt Aufsteck- bzw. Führungshülse 19 und das Griffteil 11 zusammengehalten. Wird der Handgriff 21 weiter so gedreht, daß die Zugschraube 14 über ihre reine Haltefunktion zusätzlich in das Formteil 13 hineinbewegt wird, wird das weichelastische Formteil im Bereich des Schraubenkopfes nach innen gezogen. Die Höhe des weichelastischen Formteils 13 wird damit verringert. Dadurch entsteht ein entsprechender Hohlraum zwischen dem weichelastischen Formteil 13 und der zugeordneten Gelenkfläche. In diesem Hohlraum bildet sich ein erhöhter Unterdruck aus, der einen sicheren Halt zwischen Setzinstrument 10 und der Gelenkkomponente erzeugt. Der Umfangsrand 22 dient einerseits einer zusätzlichen Abdichtung gegenüber der Gelenkkomponente, verhindert andererseits aber auch ein Abkippen der Gelenkkomponente vom Setzinstrument 10. Die Kippstabilität wird durch den sich auch über den Umfangsrand 22 erstreckenden Stützflansch 18 erhöht.

Hervorzuheben ist noch, daß bei dem beschriebenen Setzinstrument keine Teile über die zu handhabende Gelenkkomponente, z.B. Pfanne oder Inlay, seitlich vorstehen. Es ist im Bereich des Operations-Feldes sehr schlank und behindert auch nicht die Sicht des Operateurs. Der erwähnte Umfangsrand 22 samt zugeordnetem Stützflansch 18 verhindert ein Verkippen. Das Implantat kann dementsprechend achsgenau eingesetzt werden. Der Befestigungsgrad der Gelenkkomponente am Instrument ist sehr hoch. Das Instrument kann intraoperativ entfernt und wieder neu angesetzt werden. Durch die axiale Stabilität kann am Instrument auch eine Vorrichtung für eine Pfannennavigation befestigt werden. Insbesondere erlaubt das beschriebene Instrument auch eine Einhand-Bedienung.

Von Bedeutung ist auch noch eine Anordnung für ein Instrument 10 bestehend aus einem
- Satz von weichelastischen Formteilen 13 unterschiedlicher Größe entsprechend unterschiedlich großer Gelenkflächen bzw. Gelenkkomponenten, einem
- diesem Satz von unterschiedlich großen Formteilen 13 gemeinsamen Griffteil 11 sowie Zugelement, nämlich Zugschraube 14, und ggf. einem
- Satz von den unterschiedlich großen Formteilen 13 angepaßten Stützflanschen 18.

Die vorgenannte Anordnung wird dann als sogenanntes "Instrumenten-kir" zur Verfügung gestellt.

Wie Fig. 3 zeigt, kann der Handgriff 21 am proximalen Ende des stabförmigen Abschnitts 20 des Griffteils 11 aufgeschraubt oder aufgepreßt sein.

### Bezugszeichen:

- 10: Setzinstrument
- 11: Griffteil
- 12: Saugelement
- 13: weichelastisches Formteil
- 14: Zugschraube
- 15: Gewindeschaft
- 16: Schraubenkopf
- 17: Innengewinde
- 18: Stützflansch
- 19: Aufsteck- bzw. Führungshülse
- 20: stabförmiger Abschnitt
- 21: Handgriff
- 22: Umfangsrand

## Patentansprüche

1. Instrument zur Handhabung einer eine konkave Gelenkfläche umfassenden Gelenkkomponente einer Gelenkprothese, insbesondere Hüft- oder Schulterpfanneninlay-Setzinstrument (10), mit einem Griffteil (11) und einem an die Gelenkfläche abdichtend anlegbaren Saugelement (12), wobei das Saugelement (12) ein weichelastisches Formteil ist, welches durch ein Zugelement (14) derart verformbar ist, dass bei Anlage an die Gelenkfläche zwischen dieser und dem weichelastischen Formteil (13) ein, insbesondere erhöhter, Unterdruck entsteht, **dadurch gekennzeichnet, dass** das Formteil eine konvexe Oberfläche, die der Gelenkfläche angepasst ist, aufweist.

2. Instrument nach Anspruch 1,
**dadurch gekennzeichnet, daß**
das Zugelement (14) vom Griffteil (11) her manipulierbar ist.

3. Instrument nach Anspruch 1 oder 2,
**dadurch gekennzeichnet, daß**
das weichelastische Formteil (13) einen hemisphärischen Saugabschnitt aufweist.

4. Instrument nach einem der Ansprüche 1 bis 3,
**dadurch gekennzeichnet, daß**
das Zugelement eine Zugschraube (14) mit Gewindeschaft (15) und Schraubenkopf (16) umfaßt, der bei hemisphärischem Saugabschnitt des weichelastischen Formteils (13) dem Pol desselben zugeordnet ist.

5. Instrument nach Anspruch 4,
**dadurch gekennzeichnet, daß**
der Gewindeschaft (15) der Zugschraube (14) in das distale Ende des Griffteils (11) einschraubbar ist, wobei dann das weichelastische Formteil (13) zwischen Schraubenkopf (16) und Griffteil (11) bzw. einer am distalen Ende desselben angeordneten Stützfläche eingespannt ist.

6. Instrument nach einem der Ansprüche 1 bis 5,
**dadurch gekennzeichnet, daß**
das Griffteil (11) einen stabförmigen Abschnitt (20) aufweist, an dessen distalem Ende das weichelastische Formteil (13) abgestützt ist und dessen proximales Ende einen Handgriff (21) umfaßt.

7. Instrument nach Anspruch 6,
**dadurch gekennzeichnet, daß**
der stabförmige Abschnitt (20) des Griffteils (11) entweder starr oder zumindest bereichsweise biegeelastisch, z.B. nach Art einer flexiblen Antriebswelle, ausgeführt ist.

8. Instrument nach einem der Ansprüche 1 bis 7,
**dadurch gekennzeichnet, daß**
das weichelastische Formteil (13) einen sich äquatorial und radial nach außen erstreckenden Umfangsrand (22) aufweist.

9. Instrument nach einem der Ansprüche 1 bis 8,
**dadurch gekennzeichnet, daß**
am distalen Ende des Griffteils (11) ein, insbesondere relativ zu diesem drehbar gelagerter Stützflansch (18) angeordnet ist, gegen den das weichelastische Formteil (13) abstützbar ist.

10. Instrument nach Anspruch 9,
**dadurch gekennzeichnet, daß**
der Stützflansch (18) sich im wesentlichen über die gesamte proximale bzw. dem Griffteil (11) zugewandte Fläche des weichelastischen Formteils (13) erstreckt.

11. Instrument nach einem der Ansprüche 1 bis 10,
**dadurch gekennzeichnet, daß**
das Zugelement (14) drehfest am weichelastischen Formteil (13) verankert ist.

12. Instrument nach Anspruch 11,
**dadurch gekennzeichnet, daß**
das Zugelement (14) im weichelastischen Formteil (13) eingegossen ist.

13. Instrument nach einem der Ansprüche 1 bis 3 und 6 bis 12,
**dadurch gekennzeichnet, daß**
das Zugelement (14) mit einem am Griffteil (11) angeordneten Betätigungsmittel, z.B. Betätigungshebel mit Exzenter od. dgl. gekoppelt ist.

14. Instrument nach einem der Ansprüche 1 bis 13,
**dadurch gekennzeichnet, daß**
das weichelastische Formteil (13) aus Silikon od. dgl. Material besteht.

15. Anordnung für ein Instrument nach einem der Ansprüche 1 bis 14, bestehend aus einem
- Satz von weichelastischen Formteilen (13) unterschiedlicher Größe entsprechend unterschiedlich großer Gelenkflächen bzw. Gelenkkomponenten, einem
- diesem Satz von unterschiedlich großen weichelastischen Formteilen (13) gemeinsamen Griffteil (11) sowie Zugelement, insbesondere Zugschraube (14), und ggf. einem
- Satz von den unterschiedlich großen weichelastischen Formteilen (13) angepaßten Stützflanschen (18).

## Claims

1. Instrument for handling a joint component of a joint prosthesis comprising a concave articular surface, in particular a hip or shoulder socket inlay setting instrument (10), having a grip part (11) and a suction element (12) which is sealingly engageable with the joint surface, wherein the suction element (12) is a soft-elastic molded part (13), which is deformable by a tension member (14) such that when applied to the articular surface, a particularly increased vacuum is produced between the articular surface and the soft-elastic molded part (13), **characterized in that** the molded part comprises a convex surface adapted to the joint surface.

2. Instrument according to Claim 1,
**characterized in that**
the tension member (14) can be manipulated by the grip part (11).

3. Instrument according to Claims 1 or 2,
**characterized in that**
the soft-elastic molded part (13) comprises a hemispherical suction portion.

4. Instrument according to one of Claims 1 to 3,
**characterized in that**
the tension member comprises a tension bolt (14) with a threaded shank (15) and a bolt head (16), which is associated with the pole of the soft-elastic molded part (13) when the suction portion of the soft-elastic molded part (13) is hemispherical.

5. Instrument according to Claim 4,
**characterized in that** the threaded shank (15) of the tension bolt (14) can be screwed into the distal end of the grip part, wherein the soft-elastic molded part (13) then being clamped between the bolt head (16) and the grip part (11) or a supporting surface arranged at the distal end thereof.

6. Instrument according to one of Claims 1 to 5,
**characterized in that** the grip part (11) comprises a rod-shaped portion (20), at the distal end of which the soft-elastic molded part (13) is supported and the proximal end of which comprises a handle (21).

7. Instrument according to Claim 6,
**characterized in that** the rod-shaped portion (20) of the grip part (11) is either rigid or at least flexurally elastic in some areas, e.g. in the form of a flexible drive shaft.

8. Instrument according to one of Claims 1 to 7,
**characterized in that** the super-soft molded part (13) comprises a circumferential edge (22) extending equatorially and radially outwards.

9. Instrument according to one of Claims 1 to 8,
**characterized in that** a support flange (18) is arranged at the distal end of the grip part (11), in particular a support flange (18) which is mounted rotatably relative to the grip part (11) and against which the soft-elastic molded part (13) can be supported.

10. Instrument according to Claim 9,
**characterized in that** the support flange (18) extends substantially over the entire proximal surface or the surface facing the grip part (11) of the soft-elastic molded part (13).

11. Instrument according to one of Claims 1 to 10,
**characterized in that** the tension member (14) is non-rotatably fixed to the soft-elastic molded part.

12. Instrument according to Claim,
**characterized in that** the tension member (14) is cast in the soft-elastic molded part (13).

13. Instrument according to on the Claims 1 to 3 and 6 to 12,
**characterized in that** the tension member (14) is coupled to an actuating means, e.g. an actuating lever with eccentric or the like, arranged at the grip part.

14. Instrument according to one of Claims 1 to 13,
**characterized in that** the soft-elastic molded part (13) consists of silicone or similar material.

15. Arrangement for an instrument according to one of Claims 1 to 14, consisting of
- a set of super-elastic molded parts (13) of different sizes corresponding to differently sized joint surfaces or joint components,
- a grip part (11) common to this set of soft-elastic molded parts (13) of different sizes, as well as a tension member, in particular a tension bolt (14), and, if required, a
- set of the support flanges (18) adapted to the different sizes of soft-elastic molded parts (13).

## Revendications

1. Instrument, destiné à manipuler une composante d'articulation comprenant une surface d'articulation concave d'une prothèse d'articulation, notamment instrument de mise en place (10) d'une incrustation de hanche ou de cavité glénoïde, pourvu d'une partie formant manche (11) et d'un élément aspirant (12) susceptible d'être placé, de manière à assurer l'étanchéité sur la surface d'articulation, l'élément aspirant (12) comportant une pièce moulée élastique souple, qui par l'intermédiaire d'un élément de traction (14) est déformable de telle sorte que lors de son appui sur la surface d'articulation, il se créé une dépression, notamment élevée entre celui-ci et la pièce moulée (13) élastique souple, **caractérisé en ce que** la pièce moulée comporte une surface convexe, qui est adaptée à la surface d'articulation.

2. Instrument selon la revendication 1, **caractérisé en ce que** l'élément de traction (14) est manipulable à partir de la partie formant manche (11).

3. Instrument selon la revendication 1 ou 2, **caractérisé en ce que** la pièce moulée (13) élastique souple comporte un segment aspirant hémisphérique.

4. Instrument selon l'une quelconque des revendications 1 à 3, **caractérisé en ce que** l'élément de traction comprend une vis de traction (14) pourvue d'une tige filetée (15) et d'une tête de vis (16), qui dans le cas d'un segment aspirant hémisphérique de la pièce moulée (13) élastique souple est associée au pôle de cette dernière.

5. Instrument selon la revendication 4, **caractérisé en ce que** la tige filetée (15) de la vis de traction (14) est susceptible d'être vissée dans l'extrémité distale de la partie formant manche (11), la pièce moulée (13) élastique souple étant alors enserrée entre la tête de vis (16) et la partie formant manche (11) ou une surface d'appui placée sur l'extrémité distale de celle-ci.

6. Instrument selon l'une quelconque des revendications 1 à 5, **caractérisé en ce que** la partie formant manche (11) comporte un segment en forme de tige (20) sur l'extrémité distale duquel s'appuie la pièce moulée (13) élastique souple et dont l'extrémité proximale comprend une poignée (21).

7. Instrument selon la revendication 6, **caractérisé en ce que** le segment en forme de tige (20) de la partie formant manche (11) est réalisé soit en version rigide ou élastiquement déformable au moins par endroits, par exemple à la manière d'un arbre d'entraînement flexible.

8. Instrument selon l'une quelconque des revendications 1 à 7, **caractérisé en ce que** la pièce moulée (13) élastique souple comporte un bord périphérique (22) s'étendant vers l'extérieur en direction équatoriale et radiale.

9. Instrument selon l'une quelconque des revendications 1 à 8, **caractérisé en ce que** sur l'extrémité distale de la partie formant manche (11) est placée une bride d'appui (18), logée notamment en étant rotative par rapport à celle-ci, contre laquelle peut s'appuyer la pièce moulée (13) élastique souple.

10. Instrument selon la revendication 9, **caractérisé en ce que** la bride d'appui (18) s'étend sensiblement sur l'ensemble de la surface proximale ou qui fait face à la partie formant manche (11) de la pièce moulée (13) élastique souple.

11. Instrument selon l'une quelconque des revendications 1 à 10, **caractérisé en ce que** l'élément de traction (14) est ancré de manière solidaire en rotation sur la pièce moulée (13) élastique souple.

12. Instrument selon la revendication 11, **caractérisé en ce que** l'élément de traction (14) est coulé dans la pièce moulée (13) élastique souple.

13. Instrument selon l'une quelconque des revendications l à 3 et 6 à 12, **caractérisé en ce que** l'élément de traction (14) est couplé avec un moyen de manoeuvre placé sur la partie formant manche (11), par exemple un levier de manoeuvre avec excentrique ou similaire.

14. Instrument selon l'une quelconque des revendications 1 à 13, **caractérisé en ce que** la pièce moulée (13) élastique souple est en silicone ou en une matière similaire.

15. Agencement, destiné à un instrument selon l'une quelconque des revendications 1 à 14, constitué
- d'un jeu de pièces moulées (13) élastiques souples de différentes tailles, correspondant à des surfaces d'articulations ou des composantes d'articulations de différentes dimensions,
- d'une partie formant manche (11) commune audit jeu de pièces moulées (13) élastiques souples de différentes tailles ainsi qu'élément de traction, notamment vis de traction (14) et le cas échéant
- d'un jeu de brides d'appui (18) adapté aux pièces moulées (13) élastiques souples de différentes tailles.
